# EUROPÄISCHE PATENTSCHRIFT

(11) **EP 0 804 280 B1**
(45) Veröffentlichungstag und Bekanntmachung des Hinweises auf die Patenterteilung: **21.03.2007**
(21) Anmeldenummer: 96938108.6
(22) Anmeldetag: 06.11.1996
(51) Int. Cl.: B01F 17/00, A61K 8/00

(54) **EMULGATOREN**
EMULSIFYING AGENTS
EMULSIFIANTS

(30) Priorität: 15.11.1995 DE 19542572; 05.09.1996 DE 19636039
(43) Veröffentlichungstag der Anmeldung: 05.11.1997
(73) Patentinhaber: Cognis IP Management GmbH, 40589 Düsseldorf (DE)
(72) Erfinder: ANSMANN, Achim, D-40699 Erkrath (DE); KAWA, Rolf, D-40789 Monheim (DE); NITSCHE, Michael, D-42655 Solingen (DE); GONDEK, Helga, D-40589 Düsseldorf (DE)
(74) Vertreter: Fiesser, Gerold Michael
(86) Internationale Anmeldenummer: PCT/EP1996/004840
(87) Internationale Veröffentlichungsnummer: WO 1997/018033

(56) Entgegenhaltungen:
- WO-A-95/13863
- DE-A- 4 006 841
- DE-A- 4 312 009
- US-A- 5 385 750

## Beschreibung

### Gebiet der Erfindung

Die Erfindung betrifft die Verwendung von Mischungen mit definierten Gehalten an Alkylglykosiden und Alkoholen, als Emulgatoren zur Herstellung von kosmetischen und/oder pharmazeutischen Zubereitungen.

### Stand der Technik

Aus der Europäischen Patentschrift **EP-B1 0 553 241** (SEPPIC) ist die Verwendung von Mischungen aus 10 bis 40 Gew.-% Alkyloligoglucosiden, 60 Bis 90 Gew.-% Fettalkoholen und gegebenenfalls Polyglucose zur Herstellung von Emulsionen bekannt. Auch gemäß der Lehre der internationalen Patentanmeldung WO 92/07543 (Henkel) lassen sich Alkyloligoglucoside zusammen mit Fettalkoholen und Partialglyceriden als kosmetische Emulgatoren einsetzen. Es zeigt sich jedoch, daß zur Herstellung von hochviskosen O/W-Cremes die Glucosid/Fettalkohol-Emulgatoren des Stands der Technik in relativ hohen Mengen eingesetzt werden müssen. Hohe Wachskonzentrationen, d.h. hohe Mengen der Emulgatormischung, führen aber zu einer unerwünschten Belastung der Haut, insbesondere dann, wenn sensorisch leichte Produkte gewünscht werden.

Die Aufgabe der Erfindung hat somit darin bestanden, eine Mischung zur Verwendung als Emulgatoren zur Herstellung von kosmetischen und pharmazeutischen Zubereitungen zur Verfügung zu stellen, die gegenüber den Produkten des Stands der Technik die Herstellung stabiler, aber gleichzeitig sensorisch leichter Produkte mit einer verminderten Menge an Wachskörpern erlauben.

### Beschreibung der Erfindung

Gegenstand der.Erfindung ist die Verwendung von Mischungen enthaltend
(a) 43 bis 99 Gew.-% Alkyl- und/oder Alkenyloligoglykoside der allgemeinen Formel I

   R¹O-[G]ₚ (I)

   worin R¹ für einen gegebenenfalls hydroxysubstituierten Alkyl- und/oder Alkenylrest mit 16 bis 54 Kohlenstoffatomen, G für einen Zuckerrest mit 5 oder 6 Kohlenstoffatomen und p für Zahlen von 1 bis 10 steht,
   und
(b) 1 bis 57 Gew.-% Fettalkohole der allgemeinen Formel II

   R²OH (II)

   worin R² für einen aliphatischen, linearen oder verzweigten, gegebenenfalls hydroxysubstituierten Kohlenwasserstoffrest mit 16 bis 54 Kohlenstoffatomen und 0 und/oder 1, 2 oder 3 Doppelbindungen steht,
mit der Maßgabe, daß sich die Komponenten zu 100 Gew.-% addieren, als Emulgatoren zur Herstellung von kosmetischen oder pharmazeutischen Zubereitungen. Überraschenderweise wurde gefunden, daß Mischungen aus Alkylglykosiden und Fettalkoholen, die die beiden Komponenten gerade im umgekehrten Gewichtsverhältnis wie bekannte Produkte des Stands der Technik enthalten, nicht nur ein vergleichbar gutes Emulgiervermögen besitzen, sondern sich zudem mit vergleichbaren Emulgatormengen O/W-Emulsionen einer signifikant höheren Viskosität herstellen lassen. Umgekehrt lassen sich O/W-Emulsionen vergleichbarer Viskosität schon mit geringeren Mengen der neuen Emulgatoren herstellen, so daß infolge der verminderten Menge an Wachskörpern die gewünschten sensorisch leichteren Produkte erhalten werden können. Die Erfindung schließt die Erkenntnis mit ein, daß besonders viskose und sensorisch vorteilhafte Emulsionen hoher Lagerstabilität unter Mitverwendung von hydrophilen Wachsen, vorzugsweise Fettsäurepartialglyceriden erhalten werden.

### Alkyl- und/oder Alkenyloligoglykoside

Alkyl- und Alkenyloligoglykoside stellen bekannte nichtionische Tenside dar, die der Formel **(I)** folgen,

**R¹O-[G]ₚ** **(I)**

in der R¹ für einen gegebenenfalls hydroxysubststuierten, linearen oder verzweigten Alkyl- und/oder Alkenylrest mit 4 bis 54 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften **EP-A1-0 301 298** und **WO 90/03977** verwiesen. Die Alkyl- und/oder Alkenyloligoglykoside können sich von Aldosen bzw. Ketosen mit 5 oder 6 Kohlen-stoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vor-zugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Isotridecylalkohol, Myristylalkohol, Cetylalkohol, Isocetylalkohol, Palmoleylalkohol, Stearylalkohol, Isostearylalkohol, Ricinolalkohol, Hydroxystearylalkohol, Dihydroxystearylalkohol, Oleylalkohol, Elaidylalkohol, Petroselinylalkohol, Arachylalkohol, Gadofeylalkohol, Behenylalkohol, Erucylalkohol, Brassidylalkohol sowie deren technische Gemische, die wie oben beschrieben erhalten werden können. Ebenfalls eingesetzt werden können Guerbetalkohole mit 12 bis 36 Kohlenstoffatomen sowie technische Dimerdiol- und Trimertriol-Gemische mit 18 bis 36 bzw. 18 bis 54 Kohlenstoffatomen. Bevorzugt sind Alkyloligoglucoside auf Basis von Cetylstearylalkohol, Isostearylalkohol und/oder Guerbetalkoholen, vorzugsweise mit 12 bis 18 Kohlenstoffatomen, mit einem DP von 1 bis 3.

### Fettalkohole

Unter Fettalkoholen sind primäre aliphatische Alkohole der Formel (II) zu verstehen,

**R²OH** **(II)**

in der R² für einen aliphatischen, linearen oder verzweigten, gegebenenfalls hydroxysubstituierten Kohlenwasserstoffrest mit 6 bis 54 Kohlenstoffatomen und 0 und/oder 1, 2 oder 3 Doppelbindungen steht. Typische Beispiele sind Capronalkohol, Caprylalkohol, 2-Ethylhexylalkohol, Caprinalkohol Laurylalkohol, Isotridecylalkohol, Myristylalkohol, Cetylalkohol, Isocetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Ricinolalkohol, Hydroxystearylalkohol, Dihydroxystearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestem auf Basis von Fetten und Ölen oder Aldehyden aus der Roelen'schen Oxosynthese sowie als Monomerfraktion bei der Dimerisierung von ungesättigten Fettalkoholen anfallen. Weitere Beispiele sind die Guerbetalkohole auf Basis von Fettalkoholen mit 6 bis 18 Kohlenstoffen sowie technische Dimerdiole und Trimertriole mit 18 bis 36 bzw. 18 bis 54 Kohlenstoffatomen, die aus der Oligomerisierung und nachfolgenden Hydrierung ungesättigter Fettsäuren stammen. Bevorzugt sind technische Fettalkohole mit 16 bis 18 Kohlenstoffatomen wie beispielsweise Cetylstearylalkohol, lsostearylatkohol sowie Guerbetalkohole entsprechender Kettenlänge. Im Sinne der Erfindung ist es besonders vorteilhaft, Mischungen von Alkylofigoglucosiden und Fettalkoholen einzusetzen, die identische Alkylreste aufweisen, also beispielsweise Mischungen von Cetearyloligoglucosiden und Cetearylalkohol, Isostearyloligoglucosiden und isostearylalkohol oder Guerbetalkyloligoglucosiden und den korrespondierenden Guerbetalkoholen.

### Herstellverfahren

Zur Herstellung der erfindungsgemäßen Emulgatoren kann man beispielsweise handelsübliche wäßrige Glucosidpasten mit Fettalkoholen im gewünschten Verhältnis mischen und nach Bedarf.ganz oder vollständig entwässern. Vorzugsweise geht man jedoch von technischen Glucosid/Fettalkoholgemischen aus, wie sie bei der Herstellung von Alkyloligoglucosiden durch säurekatalysierte Acetalisierung von Glucose mit Fettalkohol im Überschuß anfallen. Diese Mischungen enthalten üblicherweise etwa 60 bis 80 Gew.-% Fettalkohol und 20 bis 40 Gew.-% Glucoside. Zur Einstellung des höheren Glucosidanteils kann man grundsätzlich nach zwei Methoden verfahren: Entweder wird der Fettalkoholanteil durch eine anschließende Behandlung im Dünnschicht- oder Fallfilmverdampfer destillativ bis auf das gewünschte Maß abgereichert oder aber man stockt den Glucosidanteil durch vorzugsweise wasserfreie Glucoside auf, die man ihrerseits aus wäßrigen Pasten durch Heißdampftrocknung oder durch Entwässerung im Flashdryer herstellen kann. Glucose und Cetylalkohol werden in an sich bekannter Weise acetalisiert, überschüssiger Fettalkohol ganz oder zum überwiegenden Teil abgetrennt und Stearylalkohol und/oder Isostearylalkohol in der gewünschten Menge zugemischt. Falls erforderlich, kann gebildete Polyglucose mit Hilfe von Membranen abgetrennt werden. Ein weiterhin bevorzugtes Verfahren zur Herstellung der Emulgatoren besteht darin, daß man Glucose oder Stärkesirup zunächst mit einem kurzkettigen Alkohol, beispielsweise Butanol oder einem Vorlauffettalkohol mit 6 bis 10 Kohlenstoffatomen, zu einem Niedrigalkylglucosid umsetzt und dieses anschließend mit dem gewünschten langkettigen Fettalkohol, vorzugsweise Cetearylalkohol, umacetalisiert. Die weitere Aufbereitung kann dann wie oben geschildert erfolgen.

### Gewerbliche Anwendbarkeit

Die Emulgatoren erlauben die Herstellung stabiler O/W-Emulsionen. Im Gegensatz zu anderen Emulgatoren des Stands der Technik, die einen höheren Fettalkohol- und einen niedrigeren Glucosidgehalt aufweisen, lassen sich beispielsweise hochviskose Cremes auch mit niedrigen Wachskonzentrationen herstellen, was zu einer signifikanten Verbesserung der Sensorik der Produkte führt. Der Gegenstand der Erfindung betrifft daher die Verwendung der Emulgatoren zur Herstellung von kosmetischen und/oder pharmazeutischen Zubereitungen.

### Hydrophile Wachse

In einer bevorzugten Ausführungsform der Erfindung werden die Emulgatormischungen zusammen mit konsistenzgebenden hydrophilen Wachsen eingesetzt. Hierbei handelt es sich um bei Raumtemperatur feste Stoffe, die über freie Hydroxylgruppen verfügen. Typische Beispiele sind Fettsäurepartialglyceride, d.h. technische Mono- und/oder Diester des Glycerins mit Fettsäuren mit 12 bis 18 Kohlenstoffatomen wie etwa Glycerinmono/dilaurat, -palmitat oder -stearat Weiterhin kommen auch Fettalkohole wie etwa technische Fettalkohole mit 12 bis 22 Kohlenstoffatomen, namentlich Cetylalkohol, Stearylalkohol oder Cetearylalkohol in Betracht. Die hydrophilen Wachse werden vorzugsweise in Mengen von 10 bis 30 und vorzugsweise 2,5 bis 10 Gew.-% - bezogen auf die Emulgatoren - eingesetzt. Vorteilhafte Mischungen aus den Emulgatorkomponenten (a) und (b) sowie hydrophilen Wachsen (c) haben z.B. folgende Zusammensetzungen:
(a) 25 bis 45 Gew.-% Alkyl- und/oder Alkenyloligoglykoside,
(b) 25 bis 45 Gew.-% Fettalkohole und
(c) 10 bis 50 Gew.-% hydrophile Wachse wie z.B. Fettsäurepartialglyceride,
wieder mit der Maßgabe, daß sich die Mengenangaben zu 100 Gew.-% ergänzen.

### Ölkörper

Die Emulgatoren eignen sich zur Herstellung von Emulsionen des O/W-Typs. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethythexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, Ester von C₆-C₂₂-Fettalkoholen und/oder Guerbetalkoholen mit aromatischen Carbonsäuren, vorzugsweise Benzoesäure, pflanzliche Öle, verzweigte primäre Alkohole, substituierte Cyclohexane, Guerbetcarbonate, Dialkylether und/oder aliphatische bzw. naphthenische Kohlenwasserstoffe sowie Siliconöle, Dimethicone oder Cyclomethicone in Betracht. Der Anteil der Ölkörper am nichtwäßrigen Anteil der Emulsionen kann 5 bis 99 und vorzugsweise 10 bis 75 Gew.-% ausmachen

### Tenside

Die mit Hilfe der Emulgatoren erhältlichen Emulsionen können als Bestandteile anionische, nichtionische, kationische und/oder amphotere bzw. zwitterionische Tenside enthalten. Typische Beispiele für **anionische Tenside** sind Alkylbenzolsulfonate, Alkansulfonate, Olefinsulfonate, Alkylethersulfonate, Glycerinethersutfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Acyltartrate, Acylglutamate, Acylaspartate, Alkyloligoglucosidsulfate, Proteintettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, Fettsäure-N-alkylglucamide, Proteinhydrolysate (insbesondere pflanzliche Produkte auf Weizenbasis), Polyolfeftsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und Esterquats, insbesondere quaternierte Fettsäuretrialkanolaminester-Salze. Typische Beispiele für **amphotere bzw. zwitterionische Tenside** sind Alkylbetaine, Alkylamidobetaine, Aminopropionate, Aminoglycinate, lmidazoliniumbetaine und Sulfobetaine.

Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen.

### Weitere Hilfs- und Zusatzstoffe

Die Emulsionen können als Hautpflegemittel, wie beispielsweise Tagescremes, Nachtcremes, Pflegecremes, Nährcreme, Bodylotions, Salben und dergleichen eingesetzt werden und als weitere Hilfs- und Zusatzstoffe Co-Emulgatoren, Kationpolymere, Silicone, Überfettungsmittel, Fette, Wachse, Stabilisatoren, biogene Wirkstoffe, Glycerin, Konservierungsmittel, Farb- und Duftstoffe enthalten.

Als **Co-Emulgatoren** kommen beispielsweise nichtionogene Tenside aus mindestens einer der folgenden Gruppen in Frage:
(b1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(b2) C_{12/18}-Fettsäuremono- und -diester von Anlagerungsprodukten von 1 bis 30 Mol Ethylenoxid an Glycerin;
(b3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b4) Anlagerungsprodukte von 15 bis 60 Mol Ethylenoxid an Ricinusöl und/oder gehärtetes Ricinusöl;
(b5) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(b6) Anlagerungsprodukte von 2 bis 15 Mol Ethylenoxid an Ricinusöl und/oder gehärtetes Ricinusöl;
(b7) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b8) Trialkylphosphate;
(b9) Wollwachsalkohole;
(b10) Polysiloxan-Polyalkyl-Polyether-Copolymere bzw. entsprechende Derivate;
(b11) Mischester aus Pentaerythrit, Fettsäuren, Citronensäure und Fettalkohol gemäß **DE-PS 11 65 574** und/oder Mischester von Fettsäuren mit 6 bis 22 Kohlenstoffatomen, Methylglucose und Polyolen, vorzugsweise Glycerin sowie
(b12) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht.

C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethytcarboxymethylglycinat. Besonders bevorzugt ist das unter der CTFA-Bezeichnung . *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyftaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, kationische Stärke, Copolymere von Diallylammoniumsalzen und Acrylamiden, quaternierte Vinylpyrrolido/Vinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), quaternierte Weizenpolypeptide, Polyethylenimin, kationische Siliconpolymere wie z.B. Amidomethicone oder Dow Coming, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der FR-A 22 52 840 sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol oder Partialglyceriden in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinytacetat-Copolymerisate, Polymere der Acrylsäurereihe, quaternäre Cellulose-Derivate, Kollagen, Hyaluronsäure bzw. deren Salze und ähnliche Verbindungen. Zur Verbesserung des Fließverhaltens können ferner **Hydrotrope** wie beispielsweise Ethanol, Isopropylalkohol, Propylenglycol oder Glucose eingesetzt werden. Als **Konservierungsmittel** eignen sich beispielsweise Phenoxyethanol, Formaldehydlösüng, Parabene, Pentandiol oder Sorbinsäure. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Emulsionen - betragen.

### Beispiele

### I. Anwendungstechnische Beispiele

Es wurden wäßrige O/W-Emulsionen hergestellt unter Verwendung von Cetearylglucosid (Verteilung C₁₆:C₁₈ = 1:1), dem korrespondierenden Cetearylalkohol, Caprylic/Capric Triglyceride (Myritol® 312, Henkel KGaA) als Ölkörper und gegebenenfalls Hydrogenated Palm Glycerides (Monomuls® 60-35, Henkel KGaA) als hydrophilem Wachs. Die Viskositäten der Mischungen wurden nach der Brookfield-Methode in einem RVT-Viskosimeter (20°C, 4 Upm, Spindel TE, mit Heliphath) bestimmt. Die Mischungen R1 bis R5 weisen ein Glucosid:Fettalkohol-Verhältnis im Bereich 8:2 bis 5:5 auf und sind erfindungsgemäß, die Mischungen R6 bis R10 weisen ein Glucosid-Verhältnis im Bereich 4:6 bis 2:8 auf und dienen dem Vergleich. Die Mengen an Wachskörpem, also die Summe aus Glucosid, Fettalkohol und gegebenenfalls Partialglycerid, betrug in allen Fällen 7,5 Gew.-% bezogen auf die Emulsionen. Die Ergebnisse sind in Tabelle 1 zusammengestellt.

**Tabelle 1**

| **Viskositäten von O/W-Cremes** | | | | | | | | | | |
|---|---|---|---|---|---|---|---|---|---|---|
| | **R1** | **R2** | **R3** | **R4** | **R5** | **R6** | **R7** | **R8** | **R9** | **R10** |
| Cetearylglucosid (CG) | 6,0 | 5,2 | 3,7 | 3,7 | 2,7 | 3,0 | 2,3 | 2,3 | 1,6 | 1,5 |
| Cetearylalkohol (CA) | 1,5 | 2,3 | 3,7 | 3,7 | 2,7 | 4,5 | 5,2 | 5,2 | 3,8 | 6,0 |
| Hydrog.Paim Glycerides | - | - | - | 1,0 | 2,1 | - | - | 1,0 | 2,1 | - |
| Capric/Caprylic Triglyceride | 16,0 | 16,0 | 16,0 | 16,0 | 16,0 | 16,0 | 16,0 | 16,0 | 16,0 | 16,0 |
| Wasser | ad 100 | | | | | | | | | |
| ***Verhältnis CG:CA*** | 8:2 | 7:3 | 5:5 | 5:5 | 5:5 | 4:6 | 3:7 | 3:7 | 3:7 | 2:8 |
| ***Viskosität [Pas]*** | 200 | 300 | 400 | 500 | 520 | 80 | 60 | 63 | 62 | 54 |

Die Versuche lassen sich wie folgt zusammenfassen:
- Die Verwendung von Emulgatoren mit einem Gewichtsverhältnis Glucosid:Fettalkohol im erfindungsgemäßen Bereich 43:57 bis 90:10, also gegenüber dem Stand der Technik hohen Glucosidanteil, erlauben tatsächlich die Herstellung von hochviskosen O/W-Emulsionen, die als Cremes eingesetzt werden können.
- Setzt man statt dessen entsprechend der Lehre des Stands der Technik die Emulgatoren im Gewichtsverhältnis Glucosid:Fettalkohol = 40:60 bis 20:80, also mit hohem Fettafkoholanteil ein, werden zwar ebenfalls stabile Emulsionen erhalten, deren Viskosität jedoch um eine Zehnerpotenz niedriger liegt.
- Ein Vergleich der erfindungsgemäßen Rezeptur R3 (Gewichtsverhältnis 50:50) mit der Vergleichsrezeptur R6 (Gewichtsverhältnis 40:60) zeigt, daß dieser Übergang kritisch ist, da zwischen diesen Verhältnissen ein Viskositätssprung stattfindet.
- Die Mitverwendung von Partialglyceriden ist für den beanspruchten Effekt nicht zwingend, sie führt jedoch zu einem weiteren Anstieg der Viskosität.

### II. Rezepturbeispiele

**Tabelle 2**

| **Anwendungsbeispiele (Prozentangaben als Gew.-%; Wasser+ Konservierurtgsmittel ad 100 %)** | | | |
|---|---|---|---|
| **Mittel** | **Komponente** | **INCl-Bezeichnung** | **Anteil %** |
| O/W-multi-purpose cream | Cetearylgtucosid | Cetearyl Polyglucose | 1,9 |
| | Cetearylalkohol | Cetearyl Alcohol | 1,9 |
| | Monomuls® 60-35 | Hydrogenated Palm Glycerides | 3,6 |
| | Cetiol® LC | Coco-Caprylate Caprate | 10,0 |
| | Cetiol® V | Decyl Oleate | 3,0 |
| | Myritol® 318 | Caprylic/Capric Triglyceride | 1,0 |
| | Baysilon® M 350 | | 0,5 |
| | Glycerin | | 3,0 |
| O/W-skin cream with Vitamin E | Cetearylglucosid | Ceterayl Polyglucose | 2,6 |
| | Cetearylalkohol | Cetearyl Alcohol | 2,6 |
| | Monomuls® 60-35 | Hydrogenated Palm Glycerides | 1,5 |
| | Cetiol® J 600 | Oleyl Erucate | 3,0 |
| | Cetiol® V | Decyl Oleate | 4,0 |
| | Cetiol® OE | Dicaprylyl Ether | 2,0 |
| | Baysilon® M 350 | | 0,5 |
| | Myritol® 318 | Caprylic/Capric Triglyceride | 4,0 |
| | Copherol® F 1300 | Tocopherol | 1,0 |
| | Glycerin | | 3,0 |
| Lotion O/W for normal skin | Cetearylglucosid | Ceterayl Polyglucose | 0,7 |
| | Cetearylalkohol | Cetearyl Alcohol | 0,7 |
| | Monomuls® 60-35 | Hydrogenated Palm Glycerides | 5,0 |
| | Cetiol® J 600 | Oleyl Erucate | 2,0 |
| | Cetiol® V | Decyl Oleate | 6,0 |
| | Cetiol® OE | Dicaprylyl Ether | 2,0 |
| | Baysilon® M 350 | | 0,5 |
| | Glycerin | | 3,0 |
| Day cream O/W for sensitive skin | Cetearylglucosid | Cetearyl Polyglucose | 1,2 |
| | Cetearylalkohol | Cetearyl Alcohol | 1,2 |
| | Monomuls® 60-35 | Hydrogenated Palm Glycrides | 4,5 |
| | Mandelöl | Almond Oil | 2,0 |
| | Cetiol® 868 | Octyl Stearate | 6,0 |
| | Isopropylpalmitat | Isopropyl Palmitate | 2,0 |
| | Baysilon® M 350 | | 0,5 |
| | Glycerin | | 3,0 |

## Patentansprüche

1. Verwendung von Mischungen enthaltend
(a) 43 bis 99 Gew.-% Alkyl- und/oder Alkenyloligoglykoside der allgemeinen Formel I
R¹O-[G]ₚ (I)
worin R¹ für einen gegebenenfalls hydroxysubstituierten Alkyl- und/oder Alkenylrest mit 16 bis 54 Kohlenstoffatomen, G für einen Zuckerrest mit 5 oder 6 Kohlenstoffatomen und p für Zahlen von 1 bis 10 steht,
und
(b) 1 bis 57 Gew.-% Fettalkohole der allgemeinen Formel II
R²OH (II)
worin R2 für einen aliphatischen, linearen oder verzweigten, gegebenenfalls hydroxysubstituierten Kohlenwasserstoffrest mit 16 bis 54 Kohlenstoffatomen und 0 und/oder 1, 2 oder 3 Doppelbindungen steht,
mit der Maßgabe, daß sich die Komponenten zu 100 Gew.-% addieren, als Emulgatoren zur Herstellung von kosmetischen oder pharmazeutischen Zubereitungen.

2. Verwendung nach Anspruch 1, **dadurch gekennzeichnet, daß** R¹ in Formel I für einen von Cetylalkohol, Isocetylalkohol, Palmoleylalkohol, Stearylalkohol, Isostearylalkohol, Ricinolalkohol, Hydroxystearylalkohol, Dihydroxystearylalkohol, Oleylalkohol, Elaidylalkohol, Petroselinylalkohol, Arachylalkohol, Gadoleylalkohol, Behenylalklhol, Erucylalkohol oder Brassidylalkohol oder deren technischen Gemischen oder von technischen Dimerdiol- oder Trimertriol-Gemischen mit 18 bis 54 Kohlenstoff atomen abgeleiteten Rest steht.

3. Verwendung nach Anspruch 1, **dadurch gekennzeichnet, daß** R² in Formel II für einen von Cetylalkohol, Isocetylalkohol, Palmoleylalkohol, Stearylalkohol, Isostearylalkohol, Ricinolalkohol, Hydroxystearylalkohol, Dihydroxystearylalkohol, Oleylalkohol, Elaidylalkohol, Petroselinylalkohol, Arachylalkohol, Gadoleylalkohol, Behenylalklhol, Erucylalkohol oder Brassidylalkohol oder deren technischen Gemischen oder von technischen Dimerdiol- oder Trimertriol-Gemischen mit 18 bis 54 Kohlenstoff atomen abgeleiteten Rest steht.

4. Verwendung nach Anspruch 1, **dadurch gekennzeichnet, daß** R¹ in Formel I für einen von Cetylstearylalkohol, oder Isostearylalkohol abgeleiteten Rest steht.

5. Verwendung nach Anspruch 1, **dadurch gekennzeichnet, daß** R² in Formel II für einen von Cetylstearylalkohol, oder Isostearylalkohol abgeleiteten Rest steht.

## Claims

1. Use of mixtures containing
(a) 43 to 99 wt% alkyl and/or alkenyl oligoglycosides of general formula I
R¹O-[G]ₚ (I)
wherein R¹ is an optionally hydroxysubstituted alkyl and/or alkenyl residue with 16 to 54 carbon atoms, G is a sugar residue with 5 or 6 carbon atoms and p is a number from 1 to 10,
and
(b) 1 to 57 wt% fatty alcohols of general formula II
R²OH (II)
wherein R² is an aliphatic, linear or branched, optionally hydroxysubstituted hydrocarbon residue with 16 to 54 carbon atoms and 0 and/or 1, 2 or 3 double bonds,
with the proviso that the components add up to 100 wt%, as emulsifiers for the manufacture of cosmetic or pharmaceutical preparations.

2. Use according to Claim 1, **characterised in that** R¹ in formula I stands for a residue derived from any of cetyl alcohol, isocetyl alcohol, palmoleyl alcohol, stearyl alcohol, isostearyl alcohol, ricinol alcohol, hydroxystearyl alcohol, dihydroxystearyl alcohol, oleyl alcohol, elaidyl alcohol, petroselinyl alcohol, arachyl alcohol, gadoleyl alcohol, behenyl alcohol, erucyl alcohol or brassidyl alcohol or their technical mixtures or from technical dimer diol or trimer diol mixtures with 18 to 54 carbon atoms.

3. Use according to Claim 1, **characterised in that** R² in formula II stands for a residue derived from any of cetyl alcohol, isocetyl alcohol, palmoleyl alcohol, stearyl alcohol, isostearyl alcohol, ricinol alcohol, hydroxystearyl alcohol, dihydroxystearyl alcohol, oleyl alcohol, elaidyl alcohol, petroselinyl alcohol, arachyl alcohol, gadoleyl alcohol, behenyl alcohol, erucyl alcohol or brassidyl alcohol or their technical mixtures or from technical dimer diol or trimer diol mixtures with 18 to 54 carbon atoms.

4. Use according to Claim 1, **characterised in that** R¹ in formula I stands for a residue derived from cetylstearyl alcohol or isostearyl alcohol.

5. Use according to Claim 1, **characterised in that** R² in formula II stands for a residue derived from cetylstearyl alcohol or isostearyl alcohol.

## Revendications

1. Utilisation de mélanges contenant:
(a) de 43 à 99 % en poids d'alkyloligoglycosides et/ou d'alkényloligoglycosides répondant à la formule générale I
R¹O-[G]ₚ (I)
dans laquelle R¹ représente un radical alkyle et/ou alkényle éventuellement hydroxysubstitué ayant de 16 à 54 atomes de carbone, G représente un résidu de sucre ayant 5 ou 6 atomes de carbone et p vaut de 1 à 10,
et
(b) de 1 à 57 % en poids d'alcools gras répondant à la formule générale II
R²OH (II)
dans laquelle R² représente un radical hydrocarboné éventuellement hydroxysubstitué, linéaire ou ramifié, ayant de 16 à 54 atomes de carbone et 0 et/ou 1, 2 ou 3 doubles liaisons,
avec la condition que les composants se complètent jusqu'à 100 % en poids, en tant qu'émulsifiants pour la production de préparations cosmétiques ou pharmaceutiques.

2. Utilisation selon la revendication 1, **caractérisée en ce que** R¹, dans la formule I, représente un radical dérivé d'un alcool cétylique, isocétylique, palmoléylique, stéarylique, isostéarylique, ricinoléique, hydroxystéarylique, dihydroxystéarylique, oléylique, élaïdylique, pétrosélinylique, arachylique, gadoléylique, béhénylique, érucylique ou brassidylique ou de leurs mélanges industriels ou de mélanges industriels de diols dimères ou triols trimères ayant de 18 à 54 atomes de carbone.

3. Utilisation selon la revendication 1, **caractérisée en ce que** R², dans la.formule II, représente un radical dérivé d'un alcool cétylique, isocétylique, palmoléylique, stéarylique, isostéarylique, ricinoléique, hydroxystéarylique, dihydroxystéarylique, oléylique, élaïdylique, pétrosélinylique, arachylique, gadoléylique, béhénylique, érucylique ou brassidylique ou de leurs mélanges industriels ou de mélanges industriels de diols dimères ou triols trimères ayant de 18 à 54 atomes de carbone.

4. Utilisation selon la revendication 1, **caractérisée en ce que** R¹, dans la formule I, représente un radical dérivé d'un alcool cétylstéarylique ou d'un alcool isostéarylique.

5. Utilisation selon la revendication 1, **caractérisée en ce que** R², dans la formule II, représente un radical dérivé d'un alcool cétylstéarylique ou d'un alcool isostéarylique.
